# EUROPÄISCHE PATENTSCHRIFT

(11) **EP 1 216 035 B1**
(45) Veröffentlichungstag und Bekanntmachung des Hinweises auf die Patenterteilung: **08.02.2006**
(21) Anmeldenummer: 00964184.6
(22) Anmeldetag: 16.09.2000
(51) Int. Cl.: A61K 9/70

(54) **WIRK- UND/ODER HILFSSTOFFE ENTHALTENDE ZUBEREITUNG MIT STEUERBARER FREISETZUNG DIESER STOFFE, SOWIE IHRE VERWENDUNG UND HERSTELLUNG**
PREPARATION CONTAINING ACTIVE INGREDIENTS AND/OR AUXILIARY AGENTS WITH A CONTROLLED RELEASE OF THESE SUBSTANCES AND THE USE AND PRODUCTION OF THE SAME
PREPARATION CONTENANT DES PRINCIPES ACTIFS ET/OU DES EXCIPIENTS, PERMETTANT LEUR LIBERATION CONTROLEE, UTILISATION ET PRODUCTION DE LADITE PREPARATION

(30) Priorität: 30.09.1999 DE 19946822
(43) Veröffentlichungstag der Anmeldung: 26.06.2002
(73) Patentinhaber: LTS LOHMANN Therapie-Systeme AG, 56626 Andernach (DE)
(72) Erfinder: VON FALKENHAUSEN, Christian, 53340 Meckenheim (DE); KRUMME, Markus, 56567 Neuwied (DE)
(74) Vertreter: Flaccus, Rolf-Dieter
(86) Internationale Anmeldenummer: PCT/EP2000/009061
(87) Internationale Veröffentlichungsnummer: WO 2001/022947

(56) Entgegenhaltungen:
- EP-A- 0 010 987
- EP-A- 0 872 234
- DE-A- 2 656 387
- US-A- 3 625 214

## Beschreibung

Die Erfindung betrifft Wirk- und/oder Hilfsstoffe enthaltende Zubereitungen zur zeitlich und/oder hinsichtlich der Dosis steuerbaren Freisetzung dieser Stoffe, wobei die Zubereitungen mindestens zwei Schichten in gerollter oder gefalteter Form enthalten gemäß Anspruch 1.

Wirkstoff enthaltende Zubereitungen beliebiger Darreichungsform geben ihren Wirkstoff in der Regel durch Diffusion bzw. Zerfall ab, woraus in der Regel eine nichtlineare Freisetzungskinetik resultiert. Ausführungformen solcher Systeme können als orale, rektale oder vaginale Darreichungsformen, erforderlichenfalls auch als Implantate appliziert werden.
Eine häufige Anforderung an die Applikationsform stellt dabei die lineare Wirkstofffreisetzung aus der Zubereitung dar. Es kann aber auch wünschenswert sein, das Freisetzungsprofil entsprechend spezifischen Anforderungen an eine Therapieform beliebig zu modulieren. Zubereitungen für solche kontrollierte oder gesteuerte, beispielsweise lineare, Wirkstoffabgabe sind zumeist kompliziert im Aufbau und teuer in der Herstellung.

Aus dem Stand der Technik sind eine Anzahl von Wirk- und Hilfsstoff enthaltende Zubereitungen insbesondere mit retardierender Freigabe der Inhaltsstoffe bekannt.
DE 43 41 442 beschreibt eine orale Darreichungsform, welche aus einer zentralen, Wirkstoff enthaltenden, nicht erodierbaren Schicht und einer weiteren, diese Schicht ummantelnden, weitgehend wirkstofffreien, erodierbaren Schicht besteht. Die Wirkstoffreisetzung erfolgt durch passive Diffusion aus der zentralen Schicht, welche dem Freisetzungsmedium mit einer definierten Fläche ausgesetzt ist. Die Verminderung der durch Diffusion pro Zeiteinheit freigesetzten Wirkstoffmenge wird durch zusätzlich aus der Mantelschicht infolge Erosion freiwerdender Kernflächen freigesetzten Wirkstoff kompensiert. Das Prinzip der Bereitstellung neuer, "unverbrauchter" Oberflächen mittels Erosion weitgehend wirkstoffreier Deckschichten ermöglicht durch gezielte Wahl von Kern- bzw. Mantelschichtgeometrien eine weitgehende Modulation der Freisetzungskinetik. Der Kern der Erfindung des vorgenannten Dokumentes umfaßt demnach das sukzessive Bereitstellen neuer Oberflächen von wirkstoffhaltigen Schichten.

US 3,625,214 beschreibt eine flächenhafte, spiralförmig aufgerollte Darreichungsform, welche zwei Schichten umfaßt, wobei die nach außen gewandte Schicht eine in Wasser lösliche, jedoch impermeable, wirkstoffreie Folie ist, welche mit einer nach innen gewickelten, wasserlöslichen und Wirkstoff enthaltenden Matrix beschichtet ist, wobei diese Matrix längs ihrer Ausdehnung ein Dickenprofil aufweisen kann.
Wenn diese Darreichungsform einer Körperflüssigkeit ausgesetzt wird, so erodiert bzw. löst sich die äußere Schicht und legt infolgedessen wirkstoffhaltiges Matrixmaterial frei. Dieses löst sich in der Körperflüssigkeit und setzt damit Wirkstoff frei. Infolge der spiralförmigen Aufwicklung werden innere Bereiche verzögert freigelegt, wodurch eine retardierende Wirkstoffreisetzung erfolgt, die durch unterschiedliche Dicken der wirkstoffhaltigen Matrix dosismoduliert ausgeprägt sein kann. Die Steuerung der Wirkstoffreisetzung erfolgt hier also zeitlich durch das Freilegen neuer Oberflächen und hinsichtlich der Dosis durch unterschiedliche Dicken der wirkstofftragenden Schicht.

DE 197 15 794 C1 beschreibt eine laminare Arzneiform und ein Verfahren zu ihrer Herstellung. Die Erfindung zur kontrollierten Wirkstoffabgabe umfaßt spiralförmig aufgerollte oder gefaltete Schichten auf einer Polymerfolie, die einen pharmazeutischen Wirkstoff enthält. Die Erfindung ist dadurch gekennzeichnet, daß die äußere, den Verdauungssäften zugängliche Oberflächen der wirkstoffhaltigen Polymerfolie im aufgerollten oder gefalteten Zustand höchstens 25% ihrer gesamten Oberfläche beträgt und die aufgerollten oder gefalteten Schichten so aneinander haften, daß die laminare Arzneiform im Freigabetest nach USP 23, Methode A, Apparat 2, bei 37°C und 50 Upm im künstlichen Magensaft ihre spiralförmig aufgerollte oder gefaltete Form über mindestens eine Stunde beibehält und wenigstens 30% des enthaltenden Wirkstoffs im aufgerollten oder gefalteten Zustand freigesetzt werden.

US 4,767,627 beschreibt eine wirkstoffliefernde Zubereitung mit verlängerter Verweilzeit im Magen, die eine flächenhafte Ausgestaltung eines erodierbaren Polymers umfaßt, welches einen darin enthaltenden Wirkstoff über eine kontrollierte, vorherbestimmbare und verlängerte Zeitdauer freisetzt.

US 4,268,497 beschreibt eine Zubereitung zur oralen Verabreichung in der Veterinärmedizin, die ein Medikament in einer erodierbaren Folie enthält. Die Folie weist eine erste Form auf, die eine orale Verabreichung ermöglicht, und eine zweite Gestalt im Magen, durch die sie zurückgehalten wird.

Ausgehend vom vorgenannten Stand der Technik liegt der Erfindung die Aufgabe zugrunde, eine Applikationsform für eine Wirkstoff enthaltende Zubereitung anzugeben, die weniger kompliziert in der Herstellung ist und mit einfachen sowie kostengünstigen Mitteln eine beliebig modulierbare Freisetzung ermöglicht.

Zur Lösung der Aufgabe wird vorgeschlagen, daß eine Zubereitung, welche die im Oberbegriff des Hauptanspruchs genannten Merkmale aufweist, erfindungsgemäß in der ersten Schicht mindestens einen Wirk- oder Hilfsstoff enthält, und daß diese Schicht zumindest abschnittsweise durchgehend ist, und daß mindestens einer der Parameter Breite und Konzentration des Wirk- bzw. Hilfsstoffs dieser Schicht nicht konstant ist. Außerdem sind die erfindungsgemäßen Zubereitungen dadurch gekennzeichnet, daß die zweite Schicht durchgehend ist und eine geringere Feuchtigkeitsdurchlässigkeit als die erste Schicht aufweist.

Danach betrifft die Erfindung eine Wirk- und/oder Hilfsstoffe enthaltende Zubereitung mit den genannten Merkmalen, zur zeitlich und/oder hinsichtlich der Dosis steuerbaren Freisetzung dieser Stoffe, wobei diese Zubereitung mindestens zwei Schichten (1, 2) in gerollter oder gefalteter Form enthält gemäß Anspruch 1.

Die Trägerschicht (1) kann entweder in ihrer gesamten Länge von einer wirkstoffhaltigen Matrixschicht (2) bedeckt sein, sie kann in Längsrichtung aber auch wirkstofffreie Bereiche aufweisen, so daß sich wirkstoffhaltige und wirkstofffreie Bereiche in Abständen abwechseln. Ferner kann die Trägerschicht in ihrer Längsrichtung auch Bereiche mit Matrixschichten aufweisen, welche unterschiedliche Wirk- und/oder Hilfsstoffe enthalten.

Besonders vorteilhaft sind Ausführungsformen, welche mindestens eine durchgehende und im wesentlichen feuchtigkeitsundurchlässige Schicht aufweisen. Auch diese Schicht kann, wenn dies vorteilhaft erscheint, Wirkstoffe oder Hilfsstoffe enthalten, oder beides zusammen.
Durch diese feuchtigkeitsundurchlässige Schicht hindurch erfolgt die Diffusion von Wasser oder Körperflüssigkeiten - und der damit verbundene Abbau der Schicht durch Erosion, Auflösen etc. - langsamer, als dies bei den wirkstoffhaltigen Schichten der Fall ist, so daß bei letzteren der Abbau der Schicht und damit die Wirkstofffreisetzung schneller eintritt.

Eine weitere Ausgestaltung sieht vor, daß wenigstens eine der Schichten(2) des Laminats in Körperflüssigkeit löslich beziehungsweise erodierbar ist, und eine andere Schicht (1) weniger gut löslich oder schwerer erodierbar, oder sogar unlöslich beziehungsweise nicht erodierbar ist.

Dabei ist zu beachten, daß zwischen Löslichkeit bzw. Abbaubarkeit einerseits und der Dicke des Materials andererseits eine Wechselbeziehung besteht. So kann ein weitgehend unlösliches Material verhältnismäßig dünn ausgestaltet sein, während andererseits bei der Verwendung von feuchtigkeitsdurchlässigen, leichter löslichen, erodierbaren oder bioabbaubaren Materialien die Schichten entsprechend dicker sein müssen.

Üblicherweise ist die Wirkstoffkonzentration im Längenverlauf der wirkstoffhaltigen Schicht überall gleich groß. Jedoch kann es von Vorteil sein, wenn entsprechend einer bevorzugten Ausführungsform der Erfindung die Konzentration des Wirkstoffs bzw. der Wirkstoffe in Bezug auf die Längserstreckung der wirkstoffhaltigen Schicht(en) unterschiedlich hoch ist. Die Konzentrationsunterschiede können dabei vorzugsweise in Form eines Konzentrationsgradienten ausgestaltet sein, oder in Form eines andersartig variablen Konzentrationsprofils.

Weiterhin kann von dem Merkmal Gebrauch gemacht sein, daß wenigstens eine Schicht haftklebend ist.
Die Wirkstoffschicht, auch als Matrix bezeichnet, kann über ihre gesamte Länge eine einheitliche Dicke aufweisen, wobei dann die Breite dieser Schicht über die Ausdehnung in Längsrichtung variieren kann. Daraus ergibt sich dann das sogenannte Breitenprofil.

In einer Ausführungsform der Erfindung kann bei einem aufgerollten Laminat die außen liegende Schicht wirk- und/oder hilfsstoffhaltig sein.

Es kann aber auch in einer anderen Ausführungsform eine haftklebende, flüssigkeitslösliche Wirkstoffschicht auf der Innenseite der Wicklung vorgesehen sein, so daß dadurch die weitgehend wirkstoffreie Trägerschicht eine frühzeitige Wirkstoffabgabe verhindert. Wird diese spiralförmig aufgerollte Zubereitung einer Körperflüssigkeit ausgesetzt, so löst sich der wirkstoffhaltige Kleber und entrollt das System partiell. Entsprechend der jeweils momentan freiliegenden Fläche kann dann Wirkstoff durch Diffusion oder Lösung aus der Schicht in die Körperflüssigkeit übertreten. Das Freisetzungsprofil wird damit durch die Geometrie der Wirkstoffschicht gesteuert. Dabei werden durch langsames Entrollen des Systems sukzessiv neue wirkstoffhaltige Flächen freigelegt, so daß sich das Freisetzungsprofil aus Schichtgeometrie und Entrollgeschwindigkeit ergibt.

Bei einer weiteren Ausgestaltung der Erfindung kann die Maßnahme vorgesehen sein, daß sich die wirkstoffhaltige Schicht auf der Außenseite der Spirale befindet, während die Innenwicklung durch die Trägerschicht gebildet wird.

Vorteil dieser Ausführungsform ist die Initialdosis, welche durch die wirkstoffhaltige Außenwicklung bereitgestellt wird.

Eine weitere Ausgestaltung der Erfindung sieht vor, daß Schichtbereiche mit Wirk- und/oder Hilfsstoffen vorhanden sind, die hinsichtlich ihrer Inhaltsstoffe beziehungsweise deren Löslichkeits-, Klebkraft- oder Erosionseigenschaften unterschiedlich sind.
Damit kann das Freisetzungsprofil zusätzlich weiter moduliert und insbesondere dabei dosismoduliert aufgeprägt werden. Die Steuerung der Wirkstoffreisetzung erfolgt bei dieser Ausgestaltung in zeitlich folgenden "Schüben" durch das Freilegen unterschiedlicher Oberflächen mit unterschiedlichen Wirk- und Hilfsstoffen.
Die Erfindung gestattet damit die Freisetzung unterschiedlicher Wirkstoffe mit differierender Freisetzungskinetik. Beispielsweise kann die Wirkstoffschicht durch zwei unterschiedliche Wirkstoffe tragende Bereiche gebildet sein, wobei einer dieser Bereiche eine gepulste Abgabe bereitstellt, während ein anderer Bereich eine kontinuierliche Wirkstoffabgabe ermöglicht.

Die Erfindung umfaßt weiter die Möglichkeit, daß das flächenförmig vorliegende Laminat auf einen Wickelkern aufgewickelt wird, welcher nach Beendigung der Wickelung entfernt wird, so daß eine zentrale Aussparung entsteht. Diese kann einen Durchmesser von 0,5 bis 30 mm aufweisen, bevorzugt 1 bis 10 mm, besonders bevorzugt 2 bis 5 mm.

Weiterhin kann aber auch der Wickelkern als Bestandteil der Zubereitung im System verbleiben, wobei dieser Wickelkern kompakt oder hohl, als Ring vorliegend ausgeprägt sein, einen Wirkstoff enthaltend oder weitgehend wirkstoffrei ausgeführt sein kann. Darüber hinaus kann die Breite des Wikkelkerns die maximale Breite des Laminats überschreiten. Der Durchmesser dieses Wickelkerns liegt bei 0,5 mm bis 30 mm, bevorzugt bei 1 bis 10 mm und besonders bevorzugt bei 2 bis 5 mm.

Die Wirkstoffreisetzung kann über Diffusion und/oder Lösen des Wirkstoffes aus einer in sauren und/oder basischen Milieu weitgehend unlöslichen Wirkstoffschicht erfolgen, oder durch Zerfall oder Lösung einer in sauren und/oder basischen Milieu löslichen Wirkstoffschicht.

Für eine Ausprägung eines vorgegebenen Wirkstofffreisetzungsprofils kann es von Vorteil sein, daß die Dicke einer Schicht im Bereich zwischen 1 µm und 500 µm liegt, bevorzugt zwischen 5 µm und 150 µm, besonders bevorzugt zwischen 10 µm und 30 µm. Die Breite einer wirkstoffhaltigen Schicht kann im Bereich zwischen 1 mm und 50 mm liegen, bevorzugt zwischen 1 mm und 30 mm, besonders bevorzugt zwischen 10 mm und 30 mm.
Für die Erfindung kann es weiterhin von Vorteil sein, wenn die Fläche der Wirkstoffschicht, bezogen auf die Trägerschicht, im Bereich zwischen 1 und 99% liegt, bevorzugt zwischen 10 und 80%, besonders bevorzugt zwischen 30 und 70%. Die abgewickelte Länge des Gesamtsystems kann vorteilhaft im Bereich zwischen 5 mm und 300 mm liegen, bevorzugt zwischen 10 mm und 200 mm, besonders bevorzugt zwischen 10 mm und 50 mm.

Hinsichtlich des Freisetzungsprofils sind solche Ausführungsformen der Erfindung besonders bevorzugt, welche sich durch einen linearen Freisetzungsverlauf auszeichnen. Des weiteren sind Ausführungsformen besonders bevorzugt, welche die Fähigkeit zur Freisetzung einer Initialdosis aufweisen.

Die Initialdosis kann beispielsweise durch eine wirkstoffhaltige Ausßenwicklung bereitgestellt werden.

Es kann auch von Vorteil sein, wenn die erfindungsgemäßen gerollten oder gefalteten Zubereitungen an den Seiten, die den Längsseiten der jeweiligen Schichten entsprechen, mit zusätzlichen Abdeckschichten versehen werden. Dadurch wird ein Schutz gegenüber dem Angriff von Wasser oder Körperflüssigkeiten geschaffen. Vorzugsweise enthalten diese seitlichen Abdeckschichten im wesentlichen feuchtigkeitsundurchlässige Materialien.

Zur Herstellung geeigneter Darreichungsformen werden die erfindungsgemäßen aufgerollten oder gefalteten Zubereitungen vorzugsweise in ein Substrat eingebettet, welches aus einem im sauren oder basischen Milieu löslichen Stoff bestehen kann, beispielsweise in Form von Hart- oder Weichgelatinekapseln.

Eine Verwendung der Zubereitung nach der Erfindung ist zur steuerbaren Freisetzung von Wirkstoff im Magensaftbereich vorgesehen. Sie kann jedoch auch zur steuerbaren Freisetzung von Wirkstoff im Gastrointestinaltrakt, insbesondere im Dünndarm vorgesehen sein. Ein solcher Unterschied ergibt sich in an sich bekannter Art in Abhängigkeit vom pH-Wert der Körperflüssigkeit einerseits im sauren Bereich des Magens, oder andererseits im neutralen oder basischen Bereich des Dünndarms. Vorzugsweise dient die Zubereitung zur beliebig modulierbaren Steuerung und insbesondere zur linearen Steuerung der Wirkstoffreisetzung. Schließlich kann die Freisetzung von Wirkstoff auch im Dickdarm vorgesehen sein.

Schließlich kann die Zubereitung zur steuerbaren Freisetzung von Wirkstoff und Hilfsstoff, beispielsweise in Form eines Formlings wie Zäpfchen, im Anal- oder Vaginalbereich Verwendung finden, oder auch als Implantat.

Geeignete Wirkstoffe finden sich in den Wirkstoffgruppen der Parasympatholytika (zum Beispiel Scopolamin, Atropin, Berlactyzin), der Cholinergika (z.B. Physostigmin, Nicotin), der Neuroleptika (z.B. Chlorpromazin, Halogeridol), der Monoaminoxidasehemmer (z. B. Tranylcypromin, Selegilin), der Sympathomimetika (z. B. Ephedrin, D-Norpseudoephedrin, Salbutamol, Fenfluramin), der Sympatholytika und Antisympathotonika (z. B. Propanolol, Timolol, Bupranolol, Clonidin, Dihydroergotamin, Naphazolin), der Anxiolytika (z. B. Diazepam, Triazolam), der Lokalanästhetika (z. B. Lidocain), der zentralen Analgetika (z. B. Fentanyl, Sufentanil), der Antirheumatika (z. B. Indomethacin, Piroxicam, Lornoxicam), der Koronartherapeutika (z. B. Glyceroltrinitrat, Isosorbiddinitrat), der Östrogene, Gestagene und Androgene, der Antihistaminika (z. B. Diphenhydramin, Clemastin, Terfenadin), der Prostaglandinderivate, der Vitamine (z. B. Vitamin E, cholecalciferol), der Cytostatika und der herzwirksamen Glykoside wie beispielsweise Digitoxin und Digoxin.

Als Bestandteile im Grundmaterial der Wirkstoff enthaltenden Schichten können dabei Polymere wie Polyisobutylen, Ester des Polyvinylalkohols, Polyacryl-, Polymethacryl- und Polymethylmethacrylsäure und deren Derivate, Naturkautschuk, Styrol, Isopren und styrol-Butadien-Polymerisate oder Siliconpolymere, Harzbestandteile wie gesättigte und ungesättigte Kohlenwasserstoffharze, Derivate des Abietylalkohols und des β-Pinens, Weichmacher wie Phthalsäureester, Triglyceride und Fettsäuren, sowie eine Reihe weiterer, dem Fachmann bekannter Stoffe Verwendung finden.

Für die als unlöslich ausgeführten Schichten sind mehrere, insbesondere für pharmazeutische Produkte akzeptable Materialien grundsätzlich geeignet: Polyvinylalkohol, Styrol-Dien-Blockcopolymere, Polyurethane, Polyvinylchlorid, Polymethacrylate, Polyacrylat, Polymethylacrylat, Polymethylmethacrylat und Derivate, Polyolefine sowie Polyester, um nur einige Beispiele zu nennen.

Ein Verfahren zum Herstellen einer Zubereitung nach der Erfindung ist gekennzeichnet durch die im Anspruch 20 aufgezählten Arbeitsschritte. Eine Ausgestaltung des Verfahrens sieht vor, daß zur Erzielung eines gewünschten Freisetzungsprogrammes nach der Applikation Teile der Wirk- und/oder Hilfsstoffschicht in der Längserstreckung des Laminats entfernt oder zusätzlich hinzugefügt werden. Auch können dem Laminat weitere aktive Schichten hinzukaschiert werden. Schließlich sieht ein letzter Schritt des Verfahrens vor, daß die Zubereitung in ein Substrat eingebettet wird.

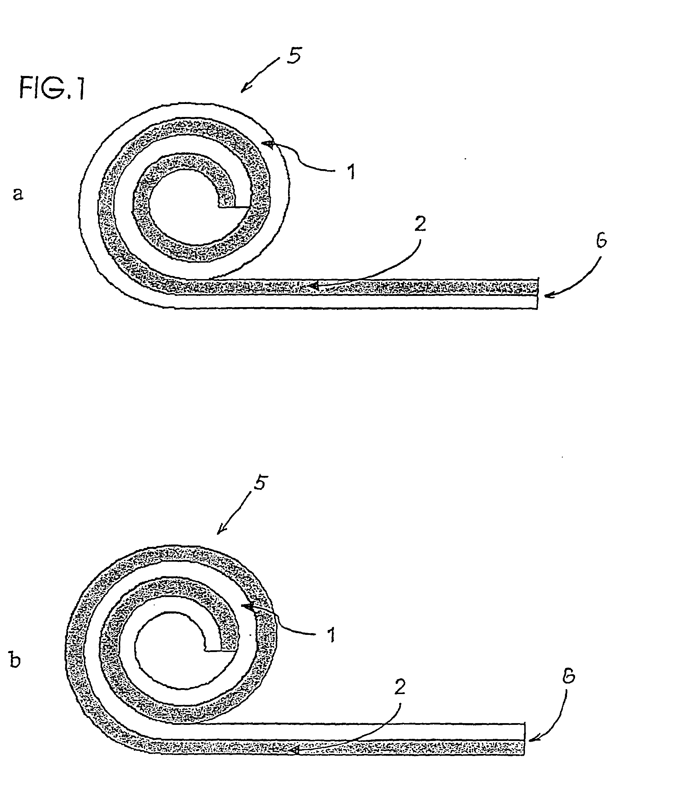

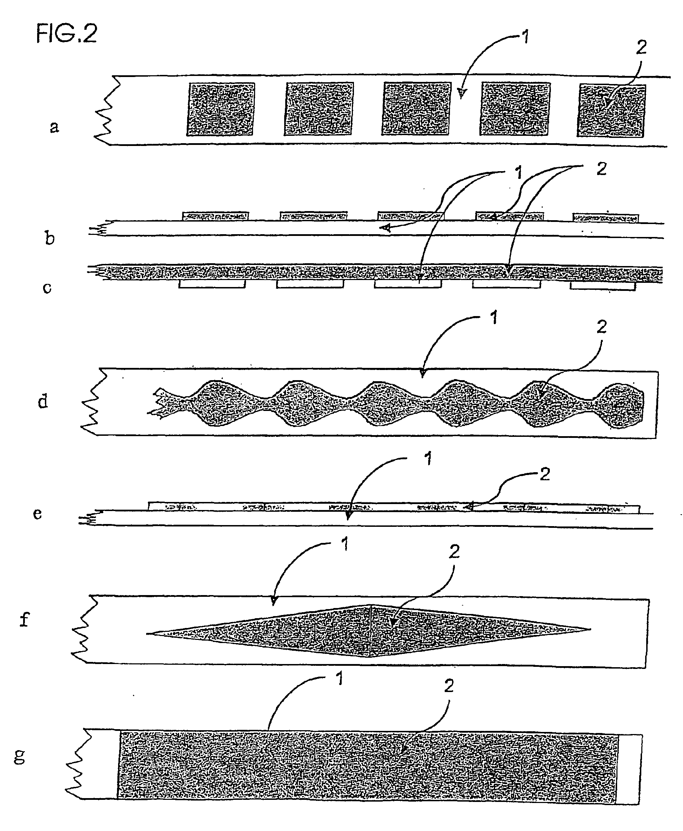

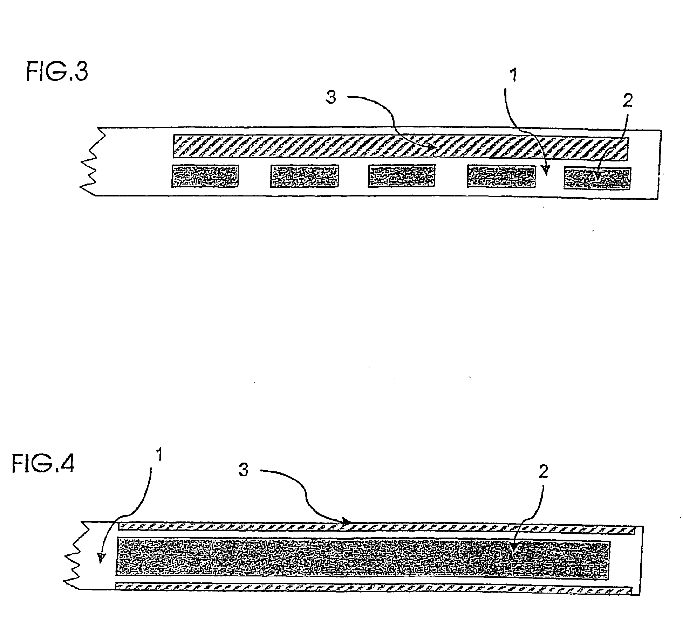

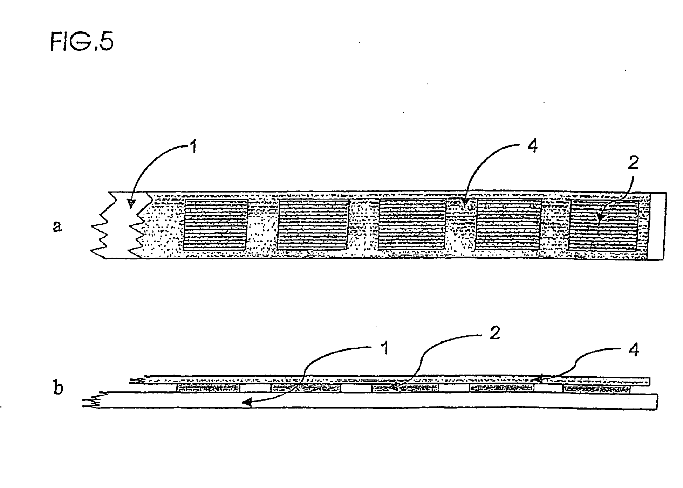

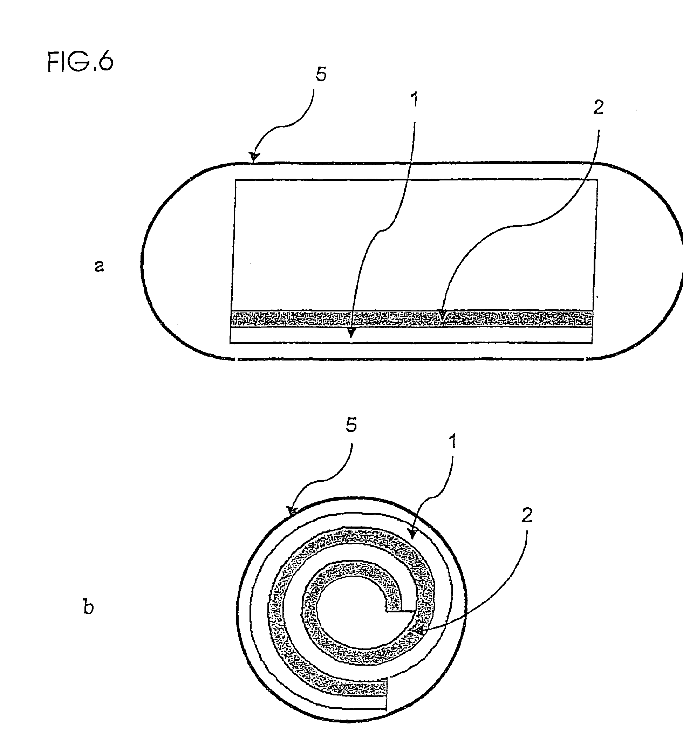

Weitere Einzelheiten, Merkmale und Vorteile der Erfindung ergeben sich aus der nachstehenden Erläuterung einiger in den Zeichnungen schematisch dargestellter Ausführungsbeispiele.
Dabei zeigen die Figuren I a/b, Figur II a-g, die Figuren III und IV, die Figur V a/b sowie die Figuren VI a/b jeweils in Seitenansicht oder in Draufsicht erfindungsgemäße Zubereitungen in einem diese Zubereitungen enthaltenden Substrats.

Die Ausführungsform gemäß Fig. Ia zeigt eine haftklebende, wasserlösliche Wirkstoffschicht (2) auf der Wicklungs-Innenseite, wobei die wirkstoffreie Trägerschicht (1) eine frühzeitige Wirkstoffabgabe verhindert.
Wird diese aufgerollte Zubereitung Körperflüssigkeiten ausgesetzt, so löst sich der wirkstoffhaltige Kleber und entrollt das System partiell. Dabei kann entsprechend der jeweils freiliegenden Fläche Wirkstoff durch Diffusion oder Lösung aus der Schicht (2) in die Körperflüssigkeit übertreten. Das Freisetzungprofil wird damit durch die Geometrie der Wirkstoffschicht gesteuert, wobei durch langsames Entrollen des Systems sukzessiv neue wirkstoffhaltige Flächen freigelegt werden und sich das Freisetzungsprofil aus Schichtgeometrie und Entrollgeschwindigkeit ergibt.

Gemäß Fig. I befindet sich die wirkstoffhaltige Schicht (2) auf der Außenseite der Spirale, während die Innenwicklung durch die Trägerschicht gebildet wird. Vorteil dieser Ausführungsform ist die Initialdosis, welche durch die wirkstoffhaltige Außenwicklung bereitgestellt wird.

Die Figuren II a, d, f, zeigen jeweils unterschiedliche Ausführungsformen des teilentrollten Systems in Draufsicht, während die Figuren II d, c, e die Ausführungsformen in Seitenansicht darstellen.
Das System gemäß Fig. II a, b ermöglicht eine zeitlich gepulste Wirkstoffabgabe, während die Ausführungsformen II d, e ein moduliertes An- bzw. Abschwellen der Abgabe zur Folge haben.
Fig. II f betrifft eine Ausprägung, welche ein langsames An- bzw. Abfluten des Wirkstoffs bereitstellt. Fig. II g zeigt eine nicht erfindungsgemäße Ausführungsform, bei welcher eine konstante Wirkstoffabgabe bereitgestellt wird, wie sie in der Pharmazie als Freisetzung nullter Ordnung bekannt ist.

Die Erfindung gestattet darüber hinaus die Freisetzung unterschiedlicher Wirkstoffe mit differierender Freisetzungskinetik. Beispielsweise zeigt Figur III eine Ausführung dieser Art, wobei die Wirkstoffschicht durch zwei unterschiedliche, Wirkstoffe tragende Bereiche (Fig. III, 2,3) gebildet wird und in diesem Fall der Bereich 2 eine gepulste Abgabe bereitstellt, während Bereich 3 eine kontinuierliche Wirkstoffabgabe ermöglicht.

Die Ausführungsform in Fig. IV umfaßt ebenfalls zwei Bereiche: Der Bereich 2 ist hierbei als wirkstoffhaltiger, wasserlöslicher Kleber ausgeführt, Bereich 3 hingegen als weitgehend wirkstoffreier oder wirkstoffhaltiger, jedoch wasserunlöslicher Dichtbereich. Hierbei übernimmt der wasserunlösliche Dichtbereich an den Rändern eine Schutzfunkton, denn ohne diese Barriere würde im ausgerollten Zustand über die seiten frühzeitig Wirkstoff freigesetzt werden. Die Stabilität der aufgerollten Zubereitung wird in diesem Fall durch den zentral aufgetragenen Kleber im Bereich 2 und nur unwesentlich durch den Barrierebereich erzeugt. Hierbei wird sichergestellt, daß Wirkstoff ausschließlich über die abgerollten, freigelegten Flächen freigesetzt wird. Vorstellbar ist aber auch, daß zu diesem Zweck die Stirnflächen eines aufgerollten Systems langsam löslich verklebt oder versiegelt werden.

Weiterhin kann von Vorteil sein, eine weitere Schicht, welche beispielsweise die haftklebenden Eigenschaften übernimmt, in das System aufzunehmen.
Fig. V a, e zeigt ein Beispiel dieser Ausführungsform. In Drauf- beziehungsweise Seitenansicht. Dabei kann diese Schicht wirkstoffhaltig oder wirkstoffrei, in Wasser löslich oder unlöslich ausgeführt sein.

Fig. VI a, b zeigt in Auf- beziehungsweise Seitenansicht die erfindungsgemäße Zubereitung in einem diese Zubereitung enthaltenden Substrat 5, welche aus einem im sauren und/oder basischen Milieu löslichen Stoff besteht.
Eine derartige Ausprägung der Erfindung kann von Vorteil sein, sofern die Trägerschicht erodierbar oder in Wasser löslich ist. Darüber hinaus kann die haftklebende Schicht unlöslich sein.
Die Figur VI zeigt das die erfindungsgemäße Zubereitung eindeckende Substrat 5 in Draufsicht (VI a) beziehungsweise in Seitenansicht (VI b). Die Zubereitung kann als Hart- beziehungsweise Weichgelatinekapsel ausgeführt sein, sie kann aber auch in Zäpfchenform vorliegen.

Die Erfindung ist unkompliziert auszuführen und löst in optimaler Weise die eingangs gestellte Aufgabe.

## Patentansprüche

1. Wirk- und/oder Hilfsstoff(e) enthaltende Zubereitung für die zeitlich und/oder hinsichtlich der Dosis steuerbare Freisetzung dieser Stoffe, enthaltend ein Laminat aus mindestens zwei Schichten (1, 2) in gerollter oder gefalteter Form,
**dadurch gekennzeichnet, daß**
a) die erste Schicht mindestens einen Wirk- oder Hilfsstoff enthält, zumindest abschnittsweise durchgehend ist und mindestens einer der Parameter Breite und Konzentration des Wirk- bzw. Hilfsstoffs dieser Schicht nicht konstant ist, und daß
b) die zweite Schicht durchgehend ist und eine geringere Feuchtigkeitsdurchlässigkeit als die erste Schicht besitzt.

2. Zubereitung nach Anspruch 1, **dadurch gekennzeichnet, daß** jeweils in Längsrichtung der Trägerschicht (1) wirkstoffhaltige Bereiche einer Matrixschicht (2) mit wirkstoffreien Bereichen der Trägerschicht (1) in Abständen abwechseln.

3. Zubereitung nach Anspruch 1 oder 2, **dadurch gekennzeichnet, daß** sie mindestens eine durchgehende und im wesentlichen feuchtigkeitsundurchlässige Schicht aufweist.

4. Zubereitung nach Anspruch 3, **dadurch gekennzeichnet, daß** die im wesentlichen feuchtigkeitsundurchlässige Schicht einen oder mehrere Wirkstoffe und / oder Hilfsstoffe enthält.

5. Zubereitung nach einem oder mehreren der vorhergehenden Ansprüche, **dadurch gekennzeichnet, daß** wenigstens eine der Schichten(2) des Laminats in Körperflüssigkeit lös-lich beziehungsweise erodierbar ist, und eine andere Schicht (1) weniger gut löslich oder schwerer erodierbar, oder sogar unlöslich beziehungsweise nicht erodierbar ist.

6. Zubereitung nach einem oder mehreren der vorhergehenden Ansprüche, **dadurch gekennzeichnet, daß** die Konzentration des Wirkstoffs bzw. der Wirkstoffe in Bezug auf die Längserstreckung der wirkstoffhaltigen Schicht(en) unterschiedlich hoch ist, vorzugsweise in Form eines Konzentrationsgradienten oder eines andersartig variablen Konzentrationsprofils.

7. Zubereitung nach einem oder mehreren der vorhergehenden Ansprüche, **dadurch gekennzeichnet, daß** wenigstens eine Schicht, insbesondere die Matrixschicht (2), haftklebend ist.

8. Zubereitung nach einem oder mehreren der vorhergehenden Ansprüche, **dadurch gekennzeichnet, daß** beim spiralförmig aufgerollten Laminat die außenliegende Schicht (2) wirk- und/oder hilfsstoffhaltig ist.

9. Zubereitung nach einem oder mehreren der Ansprüche 1 bis 7, **dadurch gekennzeichnet, daß** beim spiralförmig aufgerollten Laminat die innenliegende Schicht (2) wirk- und/oder hilfsstoffhaltig ist.

10. Zubereitung nach einem oder mehreren der vorhergehenden Ansprüche, **dadurch gekennzeichnet, daß** eine Schicht Bereiche mit Wirk- und/oder Hilfsstoffen aufweist, die sich hinsichtlich ihrer Löslichkeits-, Klebkraft- oder Erosionseigenschaften unterscheiden.

11. Zubereitung nach einem oder mehreren der vorhergehenden Ansprüche, **dadurch gekennzeichnet, daß** sie bei Ausbildung in Form eines Wickels einen Wickelkern aufweist, der wahlweise aus in Körperflüssigkeit löslichem oder unlöslichem Material besteht.

12. Zubereitung nach einem oder mehreren der vorhergehenden Ansprüche, **dadurch gekennzeichnet, daß** im Zentrum des Wickels eine röhrenförmige Aussparung von mindestens 0,5 mm Durchmesser ausgebildet ist.

13. Zubereitung nach einem oder mehreren der vorhergehenden Ansprüche, **dadurch gekennzeichnet, daß** die Zubereitung eine lineare Wirkstofffreisetzung bewirkt.

14. Zubereitung nach einem oder mehreren der Ansprüche 1 bis 12, **dadurch gekennzeichnet, daß** die Zubereitung die Freisetzung einer Initialdosis bewirkt.

15. Zubereitung nach einem oder mehreren der vorhergehenden Ansprüche, **dadurch gekennzeichnet, daß** diejenigen Seiten der spiralförmig aufgerollten oder gefalteten Zubereitung, welche den Längsseiten der jeweiligen Schichten entsprechen, mit zusätzlichen Abdeckschichten versehen sind, wobei diese Abdeckschichten vorzugsweise im wesentlichen feuchtigkeitsundurchlässige Materialien enthalten.

16. Zubereitung nach einem oder mehreren der vorhergehenden Ansprüche, **dadurch gekennzeichnet, daß** die Zubereitung in ein Substrat (5) eingebettet ist, welches vorzugsweise aus einem im sauren oder basischen Milieu löslichen Stoff besteht.

17. Zubereitung nach einem oder mehreren der Ansprüche 1 - 16 in Form einer anal oder vaginal oder als Implantat applizierbaren Darreichungsform zur Freisetzung von Wirk- und/oder Hilfsstoff.

18. Zubereitung nach einem oder mehreren der Ansprüche 1 - 16 in Form einer oral applizierbaren Darreichungsform zwecks Freisetzung von Wirk- und/oder Hilfsstoff im Gastrointestinaltrakt, insbesondere im Dünndarm oder Dickdarm.

19. Zubereitung nach einem oder mehreren der Ansprüche 1 - 16 in Form einer oral applizierbaren Darreichungsform zwecks Freisetzung von Wirk- und/oder Hilfsstoff im Magensaftbereich.

20. Verfahren zum Herstellen einer Zubereitung nach einem oder mehreren der vorhergehenden Ansprüche **gekennzeichnet durch** die Arbeitsschritte:
- Bereitstellen einer Trägerschicht (1),
- Beschichten dieser Trägerschicht (1) mit wenigstens einer Wirk- und/oder Hilfsstoff enthaltenden aktiven Schicht (2) unter Ausbildung eines Laminats,
- Trocknen des Laminats (6),
- Hinzufügen oder Entfernen von Teilen der Wirk- und/oder Hilfsstoffschicht (2) in der Längserstreckung des Laminats (6) zum Ausbilden eines Breitenprofils in der Längserstreckung der Wirk- und/oder Hilfsstoffschicht (2) des Laminats (6) zur Erzielung eines gewünschten Freisetzungsprogrammes nach der Applikation,
- Ausbilden einer Applikationsform der Zubereitung **durch** Rollen oder Falten,
- Endverpacken.

21. Verfahren nach Anspruch 20, **dadurch gekennzeichnet, daß** dem Laminat weitere aktive Schichten (3, 4) hinzukaschiert werden.

22. Verfahren nach Anspruch 20 oder 21, **dadurch gekennzeichnet, daß** die Zubereitung in ein Substrat (5) eingebettet wird.

## Claims

1. Preparation containing active and/or auxiliary substance(s), for the time- and/or dose-controllable release of said substances, comprising a laminate made up of at least two layers (1, 2) in rolled or folded shape,
**characterized**
a) in that the first layer contains at least one active or auxiliary substance, is continuous at least in sections thereof, that at least one of the parameters width and concentration of the active and/or auxiliary substance of this layer is not constant, and that
b) the second layer is continuous and possesses a lower moisture permeability than the first layer.

2. Preparation according to Claim 1, **characterized in that** in the longitudinal direction of the carrier layer (1), active substance-containing regions of a matrix layer (2) alternate at distances with active substance-free regions of the carrier layer (1).

3. Preparation according to Claim 1 or 2, **characterized in that** it comprises at least one continuous and substantially moisture-impermeable layer.

4. Preparation according to Claim 3, **characterized in that** the substantially moisture-impermeable layer contains one or more active substances and/or auxiliary substances.

5. Preparation according to one or more of the preceding Claims, **characterized in that** at least one of the layers (2) of the laminate is soluble or erodible in body fluid, and another layer (1) is less readily soluble or more difficult to erode, or is even insoluble or non-erodible.

6. Preparation according to one or more of the preceding claims, **characterized in that** the concentration of the active substance or of the active substances varies in respect of the longitudinal extension of the active substance-containing layer(s), preferably in the form of a concentration gradient or an otherwise variable concentration profile.

7. Preparation according to one or more of the preceding claims, **characterized in that** at least one layer, in particular the matrix (2), is pressure-sensitive adhesive.

8. Preparation according to one or more of the preceding claims, **characterized in that** in the spirally rolled-up laminate the outer layer (2) contains active and/or auxiliary substances.

9. Preparation according to one or more of claims 1 to 7, **characterized in that** in the spirally rolled-up laminate the inner layer (2) contains active and/or auxiliary substances.

10. Preparation according to one or more of the preceding claims, **characterized in that** one layer has regions with active and/or auxiliary substances, which regions differ in terms of their solubility, adhesive power or erosion properties.

11. Preparation according to one or more of the preceding claims, **characterized in that** if it is configured in form of a winding, it comprises a winding core which consists of material which is optionally soluble or insoluble in body fluid.

12. Preparation according to one or more of the preceding claims, **characterized in that** in the centre of the winding there is formed a tube-like recess of at least 0.5 mm in diameter.

13. Preparation according to one or more of the preceding claims, **characterized in that** the preparation effects a linear release of active substance.

14. Preparation according to one or more of claims 1 to 12, **characterized in that** the preparation effects the release of an initial dose.

15. Preparation according to one or more of the preceding claims, **characterized in that** those sides of the spirally rolled-up or folded preparation which correspond to the longitudinal sides of the respective layers are provided with additional cover layers, said cover layers preferably containing substantially moisture-impermeable materials.

16. Preparation according to one or more of the preceding claims, **characterized in that** the preparation is embedded in a substrate (5) which preferably consists of a substance that is soluble in acidic or basic environment.

17. Preparation according to one or more of claims 1 - 16, in the form of an administration form that can be administered anally or vaginally or as an implant for releasing active and/or auxiliary substance.

18. Preparation according to one or more of claims 1 - 16, in the form of an orally administrable administration form for the purpose of releasing active and/or auxiliary substances in the gastrointestinal tract, especially in the small intestine or the large intestine.

19. Preparation according to one or more of claims 1 - 16, in the form of an orally applicable administration form for the purpose of releasing active and/or auxiliary substance in the region of the gastric juice.

20. Process of manufacturing a preparation according to one or more of the preceding claims, **characterized by** the steps:
- providing a carrier layer (1),
- coating said carrier layer (1) with at least one active layer (2) containing active and/or auxiliary substance, thus forming a laminate,
- drying of the laminate (6),
- adding or removing of parts of the active substance and/or auxiliary substance layer (2) along the longitudinal extension of the laminate (6) in order to form a width profile along the longitudinal extension of the active substance and/or auxiliary substance layer (2) of the laminate (6) in order to achieve a desired release schedule following application,
- forming an application form from the preparation by rolling or folding,
- final packaging.

21. Process according to Claim 20, **characterized in that** further active layers (3, 4) are laminated to the laminate.

22. Process according to Claim 20 or 21, **characterized in that** the preparation is embedded in a substrate (5).

## Revendications

1. Préparation contenant un ou des principes actifs et/ou un ou des excipients, permettant la libération contrôlée de ces substances, en fonction du temps et/ou quant à la dose, contenant un stratifié constitué d'au moins deux couches (1, 2) sous une forme roulée ou pliée,
**caractérisée en ce que**,
a) la première couche contient au moins un principe actif ou un excipient, est au moins continue par tronçons et au moins l'un des paramètres, étendue et concentration du principe actif resp. de l'excipient de cette couche, n'est pas constant, et que
b) la deuxième couche est continue et possède une plus faible perméabilité à l'humidité que la première couche.

2. Préparation selon la revendication 1, **caractérisée en ce que**, dans la direction longitudinale de la couche porteuse (1), des zones contenant du principe actif d'une couche de matrice (2) alternent à chaque fois, par intervalles, avec des zones exemptes de principe actif de la couche porteuse (1).

3. Préparation selon la revendication 1 ou 2, **caractérisée en ce qu'**elle présente au moins une couche continue et essentiellement imperméable à l'humidité.

4. Préparation selon la revendication 3, **caractérisée en ce que** la couche essentiellement imperméable à l'humidité contient un ou plusieurs principes actifs et/ou excipients.

5. Préparation selon l'une ou plusieurs des revendications précédentes, **caractérisée en ce que**, au moins l'une des couches (2) du stratifié est soluble dans un liquide corporel respectivement peut être érodée, et une autre couche (1) est moins bien soluble ou peut être érodée plus difficilement, ou bien est même insoluble ou ne peut être érodée.

6. Préparation selon l'une ou plusieurs des revendications précédentes, **caractérisée en ce que** la concentration du principe actif resp. des principes actifs est élevée de façon différente par rapport à l'étendue longitudinale de la couche ou des couches contenant du principe actif, de préférence sous la forme d'un gradient de concentration, ou d'un profil de concentration à variabilité différente.

7. Préparation selon l'une ou plusieurs des revendications précédentes, **caractérisée en ce que**, au moins une couche, en particulier la couche de matrice (2) est auto-adhésive.

8. Préparation selon l'une ou plusieurs des revendications précédentes, **caractérisée en ce que**, dans le cas du stratifié enroulé en forme de spirale, la couche externe (2) contient du principe actif et/ou de l'excipient.

9. Préparation selon l'une ou plusieurs des revendications 1 à 7, **caractérisée en ce que**, pour le stratifié enroulé en forme de spirale, la couche interne (2) contient du principe actif et/ou un excipient.

10. Préparation selon l'une ou plusieurs des revendications précédentes, **caractérisée en ce qu'**une couche présente des zones avec des principes actifs et/ou des excipients, qui diffèrent en ce qui concerne leurs propriétés de solubilité, de force adhésive ou d'érosion.

11. Préparation selon l'une ou plusieurs des revendications précédentes, **caractérisée en ce qu'**elle présente un manchon d'enroulement lorsqu'elle est conçue sous forme de bobine, lequel est constitué facultativement d'un matériau soluble ou insoluble dans un liquide corporel.

12. Préparation selon l'une ou plusieurs des revendications précédentes, **caractérisée en ce qu'**un creux de forme tubulaire d'un diamètre d'au moins 0,5 mm est formé au centre de la bobine.

13. Préparation selon l'une ou plusieurs des revendications précédentes, **caractérisée en ce que** la préparation produit une libération linéaire de substance active.

14. Préparation selon l'une ou plusieurs des revendications 1 à 12, **caractérisée en ce que** la préparation produit la libération d'une dose initiale.

15. Préparation selon l'une ou plusieurs des revendications précédentes, **caractérisée en ce que** les côtés de la préparation enroulée ou pliée sous forme de spirale, qui correspondent aux côtés longitudinaux des couches respectives, sont munis de couches de recouvrement supplémentaires, ces couches de recouvrement contenant de préférence des matériaux essentiellement imperméables à l'humidité.

16. Préparation selon l'une ou plusieurs des revendications précédentes, **caractérisée en ce que** la préparation est incorporé dans un substrat (5), qui est constitué de préférence d'une substance soluble dans un milieu acide ou basique.

17. Préparation selon l'une ou plusieurs des revendications 1 à 16, sous la forme d'une formulation posologique applicable analement ou vaginalement, ou sous forme d'implant pour libérer un principe actif et/ou un excipient.

18. Préparation selon l'une ou plusieurs des revendications 1 à 16, sous la forme d'une formulation posologique applicable oralement, dans le but de libérer un principe actif et/ou un excipient dans le tractus gastrointestinal, en particulier dans l'intestin grêle ou le gros intestin.

19. Préparation selon l'une ou plusieurs des revendications 1 à 16, sous la forme d'une formulation posologique applicable oralement, dans le but de libérer un principe actif et/ou un excipient, dans la zone du suc gastrique.

20. Procédé pour fabriquer une préparation selon l'une ou plusieurs des revendications précédentes, **caractérisé par** les étapes opératoires :
- préparer une couche porteuse (1),
- revêtir cette couche porteuse (1) d'au moins une couche active (2) contenant un principe actif et/ou un excipient, avec formation d'un stratifié,
- sécher le stratifié (6),
- insérer ou retirer des portions de la couche de principe actif et/ou d'excipient (2) dans l'extension longitudinale du stratifié (6) pour former un profil de largeur dans l'extension longitudinale de la couche de principe actif et/ou d'excipient (2) du stratifié (6) afin de réaliser un programme souhaité de libération après l'application,
- façonner une forme d'application de la préparation par enroulement ou pliage,
- l'empaqueter finalement.

21. Procédé selon la revendication 20, **caractérisé en ce que** des couches actives (3, 4) supplémentaires sont contrecollées au stratifié.

22. Procédé selon la revendication 20 ou 21, **caractérisé en ce que** la préparation est incorporée dans un substrat (5).
